# EUROPÄISCHE PATENTANMELDUNG

(11) **EP 3 266 497 A1**
(43) Veröffentlichungstag der Anmeldung: **10.01.2018**
(21) Anmeldenummer: 17177072.0
(22) Anmeldetag: 21.06.2017
(51) Int. Cl.: A61N 1/04, A61B 17/04, A61N 1/05

(54) **ELEKTRODENFIXIERHÜLSE**

(30) Priorität: 04.07.2016 DE 102016112179
(71) Anmelder: BIOTRONIK SE & Co. KG, 12359 Berlin (DE)
(72) Erfinder: Ulmer, Jens, 8700 Küsnacht (CH)
(74) Vertreter: Keck, Hans-Georg

(57) **Zusammenfassung**

Die Erfindung betrifft eine Elektrodenfixierhülse (12) zur Fixierung einer Elektrodenleitung (14) an biologischem Gewebe, umfassend einen distalen Hülsenabschnitt (16) und einen proximalen Hülsenabschnitt (18), wenigstens einen distalen Elektrodenführungsbereich (22) an dem distalen Hülsenabschnitt (16) und wenigstens einen proximalen Elektrodenführungsbereich (24) an dem proximalen Hülsenabschnitt (18) sowie eine gemeinsame Längsachse (L) des distalen Elektrodenführungsbereichs (22) und des proximalen Elektrodenführungsbereichs (24).

Es ist vorgesehen, dass der distale Hülsenabschnitt (16) und der proximale Hülsenabschnitt (18) entlang der gemeinsamen Längsachse (L) relativ zueinander verstellbar sind und die Elektrodenfixierhülse (12) zudem wenigstens ein Elektrodenfixierelement (30) mit wenigstens anteilig elastischen Eigenschaften umfasst, das sowohl an dem distalen Hülsenabschnitt (16), als auch an dem proximalen Hülsenabschnitt (18) gelagert ist und das ausgebildet ist, sich zumindest abschnittsweise in Richtung der gemeinsamen Längsachse (L) zu verlagern, wenn entlang der gemeinsamen Längsachse (L) eine Zugkraft (Fz) auf das Elektrodenfixierelement (30) wirkt.

Weiterhin betrifft die Erfindung ein System (10) mit einer erfindungsgemäßen Elektrodenfixierhülse (12) und einer Elektrodenleitung (14).

## Beschreibung

Gegenstände der Erfindung sind eine Elektrodenfixierhülse zur Fixierung einer Elektrodenleitung an biologischem Gewebe gemäß dem Oberbegriff des Patentanspruchs 1 und ein System gemäß dem Oberbegriff des Patentanspruchs 11 mit der Elektrodenfixierhülse

### Technologischer Hintergrund und Stand der Technik

Herz-Kreislauf-Erkrankungen zählen zu den schwerwiegendsten Krankheiten der modernen Gesellschaft. Viele Erkrankungen verlaufen auch heute noch tödlich. Vor allem ältere Menschen sind von Herz-Kreislauf-Erkrankungen betroffen. Angesichts der steigenden Lebenserwartung und der wachsenden Anzahl chronischer Herzerkrankungen ist deshalb mit einer weiteren Zunahme dieser Krankheiten zu rechnen. Immer wieder angeführte Hauptursachen sind verbreitete Einflussfaktoren einer modernen und global vernetzten Gesellschaft wie Stress, Rauchen, zu fettes Essen, damit einhergehendes Übergewicht oder Bluthochdruck. Aber auch genetische Disposition oder Virusinfektionen können Herzerkrankungen verursachen. Da sich Störungen des Herz-Kreislauf-Systems bereits in jüngeren Jahren manifestieren und besonders jüngere Menschen von Beginn an in einer diese Einflussfaktoren begünstigenden Umwelt aufwachsen, ist mit einer weiteren Verstärkung des Trends zu Herz-Kreislauf-Erkrankungen zu rechnen. Einen wichtigen Ansatzpunkt stellt deshalb die konsequente Verbesserung der medizinischen Versorgung dar.

Dies führt zum einen zu steigenden Kosten und zum anderen auch zu höheren Anforderungen an die Sicherheit medizintechnischer Produkte, zum Beispiel bei Herzschrittmachern, da zukünftig tendenziell eine zunehmende Anzahl dieser Systeme im Umlauf sein wird und die Sicherheit gegen Störungen entsprechend groß sein muss.

Durch eine inhomogene Kontraktion der einzelnen Areale der linken Herzkammer kann eine Herzschwäche, auch Herzinsuffizienz genannt, entstehen. Hauptgrund für die inhomogene, beziehungsweise asynchrone Kontraktion ist eine Störung im Reizleitungssystem des Herzens. Durch die sogenannte Cardiac Resynchronization Therapy, kurz CRT-Stimulation, wird die Kontraktion der linken Herzkammer wieder homogen, vereinfacht ausgedrückt resynchronisiert, und die Pumpkraft des Herzens kann sich wieder erholen. Das Verfahren sieht die Verwendung von zwei Elektroden vor. Eine Elektrode wird in die rechte Herzkammer implantiert. Das Zielgefäß für die, die linke Herzkammer betreffende Elektrode ist der Coronarsinus. So wird das Gefäß bezeichnet, welches das venöse Blut aus den Herzkranzgefäßen in den rechten Vorhof leitet. Die Elektrode wird in einen Seitenast des Coronarsinus implantiert und liegt auf der Außenseite der linken Herzkammer. Durch die simultane Impulsabgabe über beide Elektroden wird der linke Teil des Herzens wieder synchron elektrisch erregt und eine homogene Kontraktion ist möglich.

Die Befestigung der elektrischen Leitungen an biologischem Gewebe erfolgt über sogenannte Elektrodenfixierhülsen (EFH). Darunter versteht man eine spezielle Fixierhülse, die an einer definierten Stelle fest auf der Elektrodenleitung angeordnet wird und die dann an geeigneter Stelle im menschlichen Körper befestigt wird, zum Beispiel durch Annähen im Bereich eines Muskels oder eines Gefäßes. Bei neueren Varianten werden spezielle Fixierhülsen verwendet, die zusammengepresst oder festgeschraubt werden und dann eine feste Einheit mit der Elektrode bilden. Häufig sind solche Elektrodenfixierhülsen aus einem weichen Kunststoff hergestellt, werden über die Elektrodenleitung geschoben. Mit auch Ligatur genannten Nähfaden wird dann eine Fixierungskraft auf die Elektrodenfixierhülse aufgebracht und diese dadurch an die Elektrodenleitung angepresst. Durch die entstehende Haftreibung wird die Elektrode axial auf der Elektrodenleitung fixiert. Die Elektrodenfixierhülse kann dann an geeigneter Stelle im Körper vernäht werden.

Als problematisch erweist sich hierbei, dass es bei der manuellen Anbringung der Ligatur zu einer Beschädigung der Elektrodenleitung kommen kann, wenn die aufgebrachte Fixierungskraft zu hoch ist. In einem anderen Fall kann hingegen eine ungewollte Verlagerung der Elektrodenleitung auftreten, wenn die aufgebrachte Fixierungskraft zu gering ist. Weiterhin erweist es sich als schwierig, bei Bedarf die Position der Elektrodenleitung in der Elektrodenfixierhülse zu korrigieren.

Die EP 1 933 934 B1 offenbart eine Nahthülse zum Befestigen einer implantierbaren Leitung an Körpergewebe. Die Schrift schlägt vor, mit Hilfe eines Verriegelungsmechanismus eine Beschädigung der Elektrodenleitung zu vermeiden.

Weiterhin beschreibt die US 4,553,961 eine Nahthülse mit einer Haftungsverbesserungsstruktur, die in der Nahthülse angeordnet ist und beispielsweise zusätzliche zahnartige Strukturen aufweist, mit denen die Elektrodenleitung sicher fixiert werden kann. Die Haftungsverbesserungsstruktur bildet gleichzeitig einen Schutz gegen eine zu starke Krafteinwirkung auf die Elektrodenleitung durch die Ligatur.

Die US 5,824,032 zeigt eine Verankerungshülse zur Befestigung einer Elektrodenleitung in menschlichem Gewebe. Zur Befestigung der Elektrodenleitung in der Verankerungshülse kommt eine Art Lasche zum Einsatz, die ausgebildet ist, die Elektrodenleitung zu umschlingen und durch Verriegelung der Lasche zu sichern.
Ferner offenbart die US 8,271,096 B2 eine Elektrodenfixierhülse aus einem elastischen Material mit einem darin eingeordneten formstabilen Kompressionsregler.

Der Erfindung liegt nun die Aufgabe zugrunde, eine alternative Elektrodenfixierhülse zu schaffen, mit der eine lokale Beschädigung der Elektrodenleitung vermieden wird, indem eine genau definierte Fixierungskraft gleichmäßig auf diese aufgebracht wird. Dabei soll die Fixierung der Elektrodenleitung flexibel korrigierbar sein.

Die Aufgabe wird durch die Gegenstände mit den Merkmalen der Patentansprüche 1 und 11 gelöst.

Ein erster Aspekt der Erfindung betrifft eine Elektrodenfixierhülse zur Fixierung einer Elektrodenleitung an biologischem Gewebe, umfassend einen distalen Hülsenabschnitt und einen proximalen Hülsenabschnitt sowie wenigstens einen distalen Elektrodenführungsbereich an dem distalen Hülsenabschnitt und wenigstens einen proximalen Elektrodenführungsbereich an dem proximalen Hülsenabschnitt und weiterhin eine gemeinsamen Längsachse des distalen Elektrodenführungsbereichs und des proximalen Elektrodenführungsbereichs. Erfindungsgemäß ist vorgesehen, dass der distale Hülsenabschnitt und der proximale Hülsenabschnitt entlang der gemeinsamen Längsachse relativ zueinander verstellbar sind und die Elektrodenfixierhülse zudem wenigstens ein Elektrodenfixierelement mit wenigstens anteilig elastischen Eigenschaften umfasst, das sowohl an dem distalen Hülsenabschnitt, als auch an dem proximalen Hülsenabschnitt gelagert ist und das ausgebildet ist, sich zumindest abschnittsweise in Richtung der gemeinsamen Längsachse zu verlagern, wenn entlang der gemeinsamen Längsachse eine Zugkraft auf das Elektrodenfixierelement wirkt.

Mit anderen Worten ausgedrückt bewirkt eine Längung des Elektrodenfixierelements entlang der gemeinsamen Längsachse ein Anpressen des Elektrodenfixierelements an eine Elektrodenleitung, wenn diese entlang der gemeinsamen Längsachse durch den distalen und proximalen Elektrodenführungsbereich geführt ist. Vorzugsweise erfolgt das Anpressen radial in Bezug auf die gemeinsame Längsachse. Weiterhin bevorzugt erfolgt das Anpressen von wenigstens zwei Seiten her radial in Bezug auf die gemeinsame Längsachse. Das Elektrodenfixierelement vermag gewissermaßen die Zugkraft in eine auf die Elektrodenleitung übertragbare Fixierungskraft zu wandeln. Die Fixierungskraft ist dabei radial in Bezug auf die gemeinsame Längsachse auf die Elektrodenleitung übertragbar. Grundsätzlich kann ein Fixierungsprinzip, das in Folge der Fixierungskraft Wirkung entfaltet, auf Form- und/oder Kraftschluss beruhen. Eine formschlüssige Fixierung kann beispielsweise über Halteelemente hergestellt werden, rein exemplarisch über kleine Widerhaken, die an einer Haftfläche des Elektrodenfixierungselements vorgesehen sind. Bevorzugt kommt jedoch ein kraftschlüssiges Fixierungsprinzip zur Anwendung. Hierfür ist vorgesehen, dass ein Haftschluss zwischen der Haftfläche des Elektrodenfixierelements und einer äußeren Mantelfläche der Elektrodenleitung herstellbar ist. Der Haftschluss ist herstellbar, indem der distale Hülsenabschnitt und der proximale Hülsenabschnitt voneinander wegverlagert werden. Infolge dessen wird das Elektrodenfixierelement gelängt. Die Längung ist vorzugsweise reversibel. Dies wird durch die elastischen Eigenschaften des Elektrodenfixierelements erreicht. Durch die Längung ist die Haftfläche des Elektrodenfixierelements an die Elektrodenleitung anpressbar, wenn die Elektrodenleitung in der Elektrodenfixierhülse angeordnet ist, so dass sich der Haftschluss durch Reibung ausbildet. Der Haftschluss kann gelöst werden, indem der distale Hülsenabschnitt und der proximale Hülsenabschnitt zueinander hin verlagert werden. Das Elektrodenfixierelement kehrt dann in seine ursprüngliche Form zurück und die Längung wird rückgängig gemacht. Die Haftfläche ist in diesem Zustand nicht mehr an die Elektrodenleitung angepresst und der Haftschluss ist gelöst. Die elastischen Eigenschaften des Elektrodenfixierelements können durch Wahl einer geeigneten Struktur und/oder eines elastischen Werkstoff realisiert werden. Als elastischer Werkstoff kommt beispielsweise ein elastischer Kunststoff oder ein Gummi in Betracht. Werden die elastischen Eigenschaften über eine Struktur erzeugt, so können auch Werkstoffe ohne nennenswerte eigene elastische Eigenschaften verwendet werden. Einige Beispiele für Werkstoffe sind Polyamide, Polyurethane, Polyester und deren Copolymere. Weiterhin eignen sich thermoplastische Polyolefine und Polystyrene. Ferner können auch Metalldrähte aus Nitinol oder hochlegierter Cobalt Chrom Stahl (316L, L605) oder Kohlefaser-/Kevlarfäden beispielsweise in einer Schlauchform geflochten werden. Als Grundstruktur für das Elektrodenfixierelement eignet sich beispielsweise ein Gewebe, Netz oder flächiges Vollmaterial. Die Grundstruktur kann bevorzugt räumlich zu einer Struktur, beispielsweise einer schlauchartigen Struktur, ausgeformt werden. Für die Erfindung ist es bevorzugt, dass die elastischen Eigenschaften durch Wahl einer geeigneten Struktur und eines elastischen Werkstoffs realisiert werden. Ist die Grundstruktur beispielsweise ein Netz aus elastischem Material, führt eine auf dieses Netz wirkende Zugkraft zu einer Längung in eine Richtung und zu einer Querkontraktion quer zu dieser Richtung. Wird das Netz zudem räumlich zu der Struktur angeordnet, beispielsweise schlauchartig um die gemeinsame Längsachse herum, führt eine Längung der Struktur zu einer radialen Querkontraktion um die gemeinsame Längsachse.

Die erfindungsgemäße Elektrodenfixierhülse bietet eine Reihe an Vorteilen. So kann die auf die Elektrodenleitung übertragbare Fixierungskraft über das Elektrodenfixierelement besonders homogen verteilt werden. Da auch bei elastischen Bauteilen wie dem Elektrodenfixierelement eine Längung materialbedingt und strukturbedingt begrenzt ist, ist auch die auftretende Querkontraktion begrenzt. Somit ist eine Begrenzung der Fixierungskraft möglich. Zudem ist die Fixierungskraft genau definierbar auf die Elektrodenleitung aufbringbar, da ein systematischer Zusammenhang zwischen der Fixierungskraft und der Längung des Elektrodenfixierelements besteht. All dies führt dazu, dass ein sicherer Haftschluss zwischen der Elektrodenleitung und dem Elektrodenführungsbereich herstellbar ist und eine zu starke Belastung der Elektrodenleitung vermieden werden kann. Zudem ist die Fixierungskraft reversibel auf die Elektrodenleitung übertragbar.

In bevorzugter Ausgestaltung der Elektrodenfixierhülse der Erfindung ist vorgesehen, dass das Elektrodenfixierelement wenigstens eine der gemeinsamen Längsachse zugewandte Haftfläche aufweist, die einen Reibungskoeffizienten von wenigstens 0,8 bezogen auf Kunststoffe aufweist. Die Werte können aber auch darüber und darunter liegen. Vorzugsweise handelt es sich bei dem für die Auslegung der Haftfläche konkret betrachteten Kunststoff um einen Werkstoff, der für eine Mantelschicht einer Elektrodenleitung verwendbar ist. Einige Beispiele bilden hier Silicone, Polyurethane und deren Copolymere, sowie Copolyamide (PEBAX). Weitere Beispiele sind Styrol-Blockcopolymere (SBS, SEBS, SEPS, SEEPS, MBS), Olefin basierte Elastomere sowie thermoplastische Polyester. Dies bietet den Vorteil, dass der Haftschluss zwischen der Elektrodenleitung und der Haftfläche des Elektrodenfixierelements besonders einfach und sicher realisierbar ist.

In weiterhin bevorzugter Ausgestaltung der Elektrodenfixierhülse der Erfindung ist vorgesehen, dass das Elektrodenfixierelement eine schlauchartige Struktur umfasst, deren Längsachse kollinear zu der gemeinsamen Längsachse verläuft.

Die schlauchartige Struktur eignet sich besonders gut, um eine Längung infolge der Zugkraft in eine Querkontraktion zur Aufbringung der Fixierungskraft umzusetzen. Bevorzugt wird für die schlauchartige Struktur ein flächiges Vollmaterial aus einem elastischen Material als Grundstruktur gewählt. Die schlauchartige Struktur aus dem elastischen Material kann auch direkt als Halbzeug vorliegen.

In weiterhin bevorzugter Ausgestaltung der Elektrodenfixierhülse der Erfindung ist vorgesehen, dass die schlauchartige Struktur ein definiertes Querkontraktionsverhalten aufweist, wenn die schlauchartige Struktur eine definierte Längung beziehungsweise Längsdehnung erfährt.

Die schlauchartige Struktur verringert bei einer Querkontraktion gewissermaßen ihren Durchmesser. Zur Auslegung des Querkontraktionsverhaltens wird die Verringerung des Durchmessers bezogen auf die Längsdehnung der schlauchartigen Struktur als Verhältnis herangezogen. Eine maximale Längsdehnung von 20 mm führt dabei bevorzugt zu einer maximalen Verringerung des Durchmessers von 4 mm. Das Verhältnis liegt in diesem Fall bei 5. Vorzugsweise liegt das Verhältnis im Bereich von 2 bis 4,75, weiterhin bevorzugt 4 bis 4,5. Da elastische Material- und Struktureigenschaften in der Praxis häufig nicht linear sind, wird das Verhältnis in solchen Fällen zweckmäßigerweise linear angenähert. Dem Fachmann ist ohne Weiteres klar, dass für die Auslegung eine konkrete Elektrodenleitung theoretisch zugrunde gelegt werden muss. Das Verhältnis wird dann für einen Wertebereich der Längsdehnung betrachtet und linear angenähert, innerhalb dessen ein Übergangspunkt vom Nicht-Vorliegen eines Haftschlusses zum Vorliegen eines Haftschlusses liegt.

In weiterhin bevorzugter Ausgestaltung der Elektrodenfixierhülse der Erfindung ist vorgesehen, dass die Haftfläche des Elektrodenfixierelements 30 mm² bis 200 mm² beträgt. Weiterhin bevorzugt beträgt die Haftfläche des Elektrodenfixierelements 50 mm² bis 150 mm², weiterhin bevorzugt 70 mm² bis 120 mm² und weiterhin bevorzugt 90 mm² bis 100 mm².

Die erforderliche Längsdehnung des Elektrodenfixierelements beeinflusst eine Gesamtgröße der Elektrodenfixierhülse, insbesondere eine Gesamtlänge. Da die Elektrodenfixierhülse im menschlichen Körper platziert wird, gilt es diese zu begrenzen. Untersuchungen haben gezeigt, dass in diesen Größenordnungen der Haftfläche ein sicherer Haftschluss bei einer nicht zu großen erforderlichen Längsdehnung des Elektrodenfixierelements gewährleistet ist.

Unter Beachtung der obigen Auslegungsparameter kann die Gesamtlänge der Elektrodenfixierhülse vorteilhaft auf maximal 20 mm bis 30 mm begrenzt werden. Die Gesamtlänge der Elektrodenfixierhülse beträgt vorzugsweise 23 mm bis 27 mm.
In weiterhin bevorzugter Ausgestaltung der Elektrodenfixierhülse der Erfindung ist vorgesehen, dass das Elektrodenfixierelement an einem distalen Ende und/oder an einem proximalen Ende wenigstens einen Gleitkörper umfasst, mit dem das Elektrodenfixierelement um die gemeinsame Längsachse herum drehbar und entlang der gemeinsamen Längsachse festgelegt an dem proximalen Hülsenabschnitt und/oder dem distalen Hülsenabschnitt gelagert ist.

Die Gleitkörper können beispielsweise jeweils eine Scheibe sein, die in je einer radialen Aufnahmenut in dem proximalen Hülsenabschnitt und dem distalen Hülsenabschnitt gelagert sind. Somit kann sich das Elektrodenfixierelement frei um die gemeinsame Längsachse drehen. Natürlich kann das Elektrodenfixierelement auch nur einseitig einen Gleitkörper aufweisen und anderseitig fest mit dem proximalen oder distalen Hülsenabschnitt verbunden sein.

Auf diesem Wege wird vorteilhaft gewährleistet, dass es nicht zu einer ungewünschten Querkontraktion des Elektrodenfixierelements infolge einer ungewünschten Verdrillung kommt.

In weiterhin bevorzugter Ausgestaltung der Elektrodenfixierhülse der Erfindung ist vorgesehen, dass das Elektrodenfixierelement zumindest abschnittsweise in dem distalen Elektrodenführungsbereich und dem proximalen Elektrodenführungsbereich angeordnet ist.

Dies bietet den Vorteil, dass ein Bereich für die Übertragung der Fixierungskraft auf die Elektrodenleitung vergrößert wird.

In weiterhin bevorzugter Ausgestaltung der Elektrodenfixierhülse der Erfindung ist vorgesehen, dass der proximale Hülsenabschnitt und der distale Hülsenabschnitt über ein Gewinde entlang der gemeinsamen Längsachse relativ zueinander verstellbar sind.

Dies bietet den Vorteil, dass der proximale Hülsenabschnitt und der distale Hülsenabschnitt besonders einfach und genau voneinander weg oder zueinander hin verlagerbar sind. Das Elektrodenfixierelement kann zudem sehr genau und unter einem geringen manuellen Kraftaufwand gelängt werden. Besonders vorteilhaft stellt sich diese Ausführungsform unter Verwendung jeweils eines Gleitkörpers am distalen Ende und am proximalen Ende des Elektrodenfixierelements zum Ausgleich einer Drehbewegung des Gewindes dar.

In weiterhin bevorzugter Ausgestaltung der Elektrodenfixierhülse der Erfindung ist vorgesehen, dass die Elektrodenfixierhülse ein Sicherungselement zur Sicherung eines relativen Justagezustands des distalen Hülsenabschnitts und des proximalen Hülsenabschnitts umfasst. Der Justagezustand umfasst dabei wenigstens einen Abstand des distalen Hülsenabschnitts und des proximalen Hülsenabschnitts entlang der gemeinsamen Längsachse. Er kann auch deren relative Orientierung um die gemeinsame Längsachse umfassen.

Durch das Sicherungselement wird vorteilhaft gewährleistet, dass eine eingestellte Längsdehnung des Elektrodenfixierelements sicher erhalten bleibt. Das Sicherungselement kann auch als Markierung dienen, wann der gewünschte Justagezustand erreicht ist. Somit ist sichergestellt, dass keine fehlerhafte Einstellung der Fixierungskraft erfolgt.

In weiterhin bevorzugter Ausgestaltung der Elektrodenfixierhülse der Erfindung ist vorgesehen, dass die Elektrodenfixierhülse ein Sicherungselement in Form einer Gewindeunterbrechung und/oder eines Rastelements umfasst.

Auf diesen Wegen lässt sich das Sicherungselement besonders einfach und sicher realisieren.

Ein zweiter Aspekt der Erfindung betrifft ein System, umfassend wenigstens eine erfindungsgemäße Elektrodenfixierhülse und eine Elektrodenleitung.

Die Elektrodenfixierhülse des erfindungsgemäßen Systems und die Elektrodenleitung entsprechend der obigen Beschreibung. Dies gilt auch für die oben beschriebenen konstruktiven Zusammenhänge und realisierbaren Wechselwirkungen zwischen der Elektrodenfixierhülse und der Elektrodenleitung.

Das System der Erfindung bietet den Vorteil, dass mit dem Elektrodenfixierelement der Elektrodenfixierhülse eine definierte Fixierungskraft homogen und sicher auf die Elektrodenleitung übertragen werden kann. Alle weiteren Vorteile ergeben sich im Detail aus der obigen Beschreibung.

In einer bevorzugten Ausgestaltung des Systems der Erfindung ist vorgesehen, dass ein Reibungskoeffizient zwischen dem Elektrodenfixierelement der Elektrodenfixierhülse und der Elektrodenleitung wenigstens 0,8 beträgt.

Dies bietet den Vorteil, dass der Haftschluss zwischen der Elektrodenleitung und dem Elektrodenfixierelement besonders einfach und sicher realisierbar ist.

In weiterhin bevorzugter Ausgestaltung des Systems der Erfindung ist vorgesehen, dass durch relative Verstellung des distalen Hülsenabschnitts und des proximalen Hülsenabschnitts entlang der gemeinsamen Längsachse eine entsprechende Längsdehnung der schlauchartigen Struktur des Elektrodenfixierelements erzeugbar ist und infolge des definierten Querkontraktionsverhaltens der schlauchartigen Struktur der Haftschluss zwischen der schlauchartigen Struktur und der Elektrodenleitung herstellbar ist.

So ist vorteilhaft einfach und sicher ein definierter Haftschluss zwischen der Elektrodenleitung und der schlauchartigen Struktur herstellbar.

In einer bevorzugten Ausgestaltung des Systems der Erfindung ist vorgesehen, dass der Haftschluss eine Haftreibungskraft von 2 N bis 50 N zu übertragen vermag. Bevorzugt beträgt die übertragbare Haftreibungskraft 5 N bis 25 N, weiterhin bevorzugt 7 N bis 10 N.

In einer bevorzugten Ausgestaltung des Systems der Erfindung ist vorgesehen, dass der Haftschluss durch einen relativen Verstellweg des distalen Hülsenabschnitts und des proximalen Hülsenabschnitts von 2 mm bis 20 mm herstellbar ist. Bevorzugt beträgt der Verstellweg 5 mm bis 15 mm, weiterhin bevorzugt 7 mm bis 10 mm.

Somit sind vorteilhaft eine kompakte Baugröße der Elektrodenfixierhülse und ein sicherer Haftschluss realisierbar.

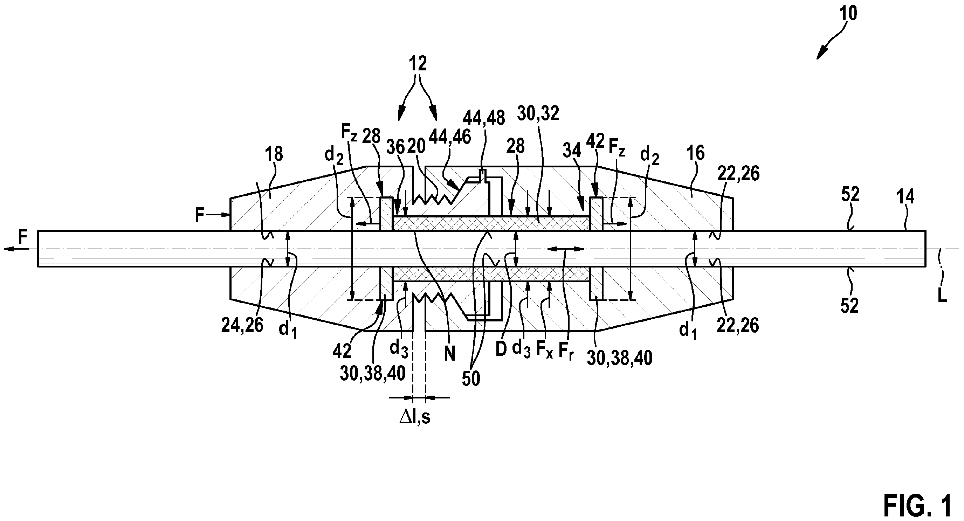

- Fig. 1: zeigt ein erfindungsgemäßes System mit einer erfindungsgemäßen Elektrodenfixierhülse und einer Elektrodenleitung in einer bevorzugten Ausführungsform.

Figur 1 zeigt ein erfindungsgemäßes System 10 mit einer erfindungsgemäßen Elektrodenfixierhülse 12 und einer Elektrodenleitung 14. Die Elektrodenfixierhülse 12 ist in einer Querschnittsansicht gezeigt.

Die erfindungsgemäße Elektrodenfixierhülse 12 weist einen distalen Hülsenabschnitt 16 und einen proximalen Hülsenabschnitt 18 auf. Der proximale Hülsenabschnitt 18 und der distale Hülsenabschnitt 16 sind über ein Gewinde 20 entlang einer gemeinsamen Längsachse L relativ zueinander verstellbar. Der distale Hülsenabschnitt 16 weist einen distalen Elektrodenführungsbereich 22 auf und der proximale Hülsenabschnitt 18 einen proximalen Elektrodenführungsbereich 24. Der proximale und distale Elektrodenführungsbereich 22, 24 sind jeweils als zylindrische Durchgangsbohrungen 26 durch den jeweiligen Hülsenabschnitt 16, 18 ausgebildet. Ein Durchmesser d₁₋₃ der zylindrischen Durchgangsbohrungen 26 variiert entlang der gemeinsamen Längsachse L. Somit ergibt sich ein Aufnahmebereich 28 für ein Elektrodenfixierungselement 30. Das Elektrodenfixierungselement 30 umfasst eine schlauchartige Struktur 32, die an jeweils einem distalen Ende 34 und einem proximalen Ende 36 einen Gleitkörper 38 aufweist. Die jeweiligen Gleitkörper 38 sind hier als Scheiben 40 ausgebildet. Die Scheiben 40 sind in Bereichen der zylindrischen Durchgangsbohrungen 26 angeordnet, die den Durchmesser d₂ aufweisen und somit je eine Aufnahmenut 42 für die Scheiben 40 bilden. In der jeweiligen Aufnahmenut 42 ist jeweils eine der Scheiben 40 um die gemeinsame Längsachse L drehbar gelagert. Die schlauchartige Struktur 32 ist in Bereichen der zylindrischen Durchgangsbohrungen 26 angeordnet, die den Durchmesser d₃ aufweisen. Über die Scheiben 40 ist auch die schlauchartige Struktur 32 um die gemeinsame Längsachse L drehbar. Die schlauchartige Struktur 32 besteht aus Silikon und weist elastische Materialeigenschaften auf. Die schlauchartige Struktur 32 ist hier außerdem als Gewebe ausgebildet. Allgemein gesprochen funktioniert die schlauchartige Struktur 32 hier nach dem Prinzip der sogenannten chinesischen Fingerfalle.

Durch Verstellung des distalen Hülsenabschnitts 16 und des proximalen Hülsenabschnitts 18 relativ zueinander entlang der gemeinsamen Längsachse L über das Gewinde 20 kann eine Zugkraft F_{z} über die Scheiben 40 auf die schlauchartige Struktur 32 aufgebracht werden. Dies führt dazu, dass die schlauchartige Struktur 32 eine Längsdehnung Δl erfährt. Die Längsdehnung Δl entspricht auch einem Verstellweg s, den der distale Hülsenabschnitt 16 relativ zu dem proximalen Hülsenabschnitt 18 entlang der gemeinsamen Längsachse L zurücklegt. Die Längsdehnung Δl beziehungsweise der Verstellweg s sind hier durch ein Sicherungselement 44 in Form einer Gewindeunterbrechung 46 begrenzt. Das Sicherungselement umfasst zudem ein Rastelement 48, durch welches sichergestellt ist, dass der distale Hülsenabschnitt 16 und der proximale Hülsenabschnitt 18 bei einem definierten Verstellweg s, der einem definierten relativen Justagezustand entspricht, in diesem verbleiben. Die schlauchartige Struktur 32 weist ein definiertes Querkontraktionsverhalten auf. Infolge der Längsdehnung Δl wird ein Durchmesser D der schlauchartigen Struktur 32 elastisch reduziert.

In dem erfindungsgemäßen System 10, das neben der Elektrodenfixierhülse 12 auch die Elektrodenleitung 14 als Element umfasst, wird durch die Verringerung des Durchmessers D eine Haftfläche 50 der schlauchartigen Struktur 32 an eine äußere Mantelfläche 52 der Elektrodenleitung 14 angepresst. Hierdurch wird eine Fixierungskraft Fₓ auf die Elektrodenleitung 14 aufgebracht. Diese führt zu einem Haftschluss zwischen der äußeren Mantelfläche 52 der Elektrodenleitung 14 und der Haftfläche 50 der schlauchartigen Struktur 32.

Vorliegend führt ein Verstellweg s beziehungsweise eine Längsdehnung Δl von 3 mm zu einer Verringerung des Durchmessers D von 0,6 mm. Aus diesen Werten ergibt sich für die Fixierungskraft Fₓ ein Wert von 20 N. Die Haftfläche 50 der schlauchartigen Struktur 32 beträgt hier 100 mm², so dass die Fixierungskraft Fₓ zu einer Flächenpressung von 0,2 N/mm² führt. Als Werkstoff der schlauchartigen Struktur 32 kommt vorliegend ein Gewebe aus Polyester, Polyamid, Nylon oder Kevlar zum Einsatz. Auch ein Mischgewebe ist möglich. Die äußere Mantelschicht 52 der Elektrodenleitung 14 besteht aus Silikon oder Silikon-Urethan-Copolymer. Zwischen der Haftfläche 50 der schlauchartigen Struktur 32 und der äußeren Mantelschicht 52 der Elektrodenleitung 14 herrscht somit ein Reibungskoeffizient µ 0,8 bis 1,2. Der Reibungskoeffizient µ kann aber auch darüber oder darunter liegen und lässt sich beispielsweise durch Auftrag eines Gleitmittels beeinflussen. Der Zuständige Fachmann legt dies anhand der offenbarten Auslegungsaspekte selbstständig fest. Infolge dessen vermag der Haftschluss ein Verrutschen der Elektrodenleitung 14 in der Elektrodenfixierhülse 12 bis zu einer auf die Elektrodenleitung 14 wirkenden Kraft F von 20 N sicher zu halten.

## Patentansprüche

1. Elektrodenfixierhülse (12) zur Fixierung einer Elektrodenleitung (14) an biologischem Gewebe, umfassend:
- einen distalen Hülsenabschnitt (16) und einen proximalen Hülsenabschnitt (18);
- wenigstens einen distalen Elektrodenführungsbereich (22) an dem distalen Hülsenabschnitt (16) und wenigstens einen proximalen Elektrodenführungsbereich (24) an dem proximalen Hülsenabschnitt (18);
- eine gemeinsame Längsachse (L) des distalen Elektrodenführungsbereichs (22) und des proximalen Elektrodenführungsbereichs (24),
**dadurch gekennzeichnet, dass**
der distale Hülsenabschnitt (16) und der proximale Hülsenabschnitt (18) entlang der gemeinsamen Längsachse (L) relativ zueinander verstellbar sind und die Elektrodenfixierhülse (12) zudem wenigstens ein Elektrodenfixierelement (30) mit wenigstens anteilig elastischen Eigenschaften umfasst, das sowohl an dem distalen Hülsenabschnitt (16), als auch an dem proximalen Hülsenabschnitt (18) gelagert ist und das ausgebildet ist, sich zumindest abschnittsweise in Richtung der gemeinsamen Längsachse (L) zu verlagern, wenn entlang der gemeinsamen Längsachse (L) eine Zugkraft (F_{z}) auf das Elektrodenfixierelement (30) wirkt.

2. Elektrodenfixierhülse (12) nach Anspruch 1, **dadurch gekennzeichnet, dass** das Elektrodenfixierelement (30) wenigstens eine der gemeinsamen Längsachse (L) zugewandte Haftfläche (50) aufweist, die einen Reibungskoeffizienten (µ) von wenigstens 0,8 bezogen auf Kunststoffe aufweist.

3. Elektrodenfixierhülse (12) nach einem der vorhergehenden Ansprüche, **dadurch gekennzeichnet, dass** das Elektrodenfixierelement (30) eine schlauchartige Struktur (32) umfasst, deren Längsachse kollinear zu der gemeinsamen Längsachse (L) verläuft.

4. Elektrodenfixierhülse (12) nach Anspruch 3, **dadurch gekennzeichnet, dass** die schlauchartige Struktur (32) ein definiertes Querkontraktionsverhalten aufweist, wenn die schlauchartige Struktur (32) eine definierte Längsdehnung (Δl) erfährt.

5. Elektrodenfixierhülse (12) nach einem der vorhergehenden Ansprüche, **dadurch gekennzeichnet, dass** die Haftfläche (50) des Elektrodenfixierelements (30) 30 mm² bis 200 mm² beträgt.

6. Elektrodenfixierhülse (12) nach einem der vorhergehenden Ansprüche, **dadurch gekennzeichnet, dass** das Elektrodenfixierelement (30) an einem distalen Ende (34) und/oder an einem proximalen Ende (36) wenigstens einen Gleitkörper (38) umfasst, mit dem das Elektrodenfixierelement (30) um die gemeinsame Längsachse (L) herum drehbar und entlang der gemeinsamen Längsachse (L) festgelegt an dem proximalen Hülsenabschnitt (18) und/oder dem distalen Hülsenabschnitt (16) gelagert ist.

7. Elektrodenfixierhülse (12) nach einem der vorhergehenden Ansprüche, **dadurch gekennzeichnet, dass** das Elektrodenfixierelement (30) zumindest abschnittsweise in dem distalen Elektrodenführungsbereich (22) und dem proximalen Elektrodenführungsbereich (24) angeordnet ist.

8. Elektrodenfixierhülse (12) nach einem der vorhergehenden Ansprüche, **dadurch gekennzeichnet, dass** der proximale Hülsenabschnitt (18) und der distale Hülsenabschnitt (16) über ein Gewinde (20) entlang der gemeinsamen Längsachse (L) relativ zueinander verstellbar sind.

9. Elektrodenfixierhülse (12) nach einem der vorhergehenden Ansprüche, **dadurch gekennzeichnet, dass** die Elektrodenfixierhülse (12) ein Sicherungselement (44) zur Sicherung eines relativen Justagezustands des distalen Hülsenabschnitts (16) und des proximalen Hülsenabschnitts (18) umfasst.

10. Elektrodenfixierhülse (12) nach Anspruch 8 oder 9, **dadurch gekennzeichnet, dass** die Elektrodenfixierhülse (12) ein Sicherungselement (44) in Form einer Gewindeunterbrechung (46) und/oder eines Rastelements (48) umfasst.

11. System (10), umfassend wenigstens:
- eine Elektrodenfixierhülse (12) nach einem der Ansprüche 1 bis 10; und
- eine Elektrodenleitung (14).

12. System (10) nach Anspruch 11, **dadurch gekennzeichnet, dass** ein Reibungskoeffizient (µ) zwischen einem Elektrodenfixierelement (30) der Elektrodenfixierhülse (12) und der Elektrodenleitung (14) bei wenigstens 0,8 liegt.

13. System (10) nach Anspruch 11 oder 12, **dadurch gekennzeichnet, dass** durch relative Verstellung des distalen Hülsenabschnitts (16) und des proximalen Hülsenabschnitts (18) entlang der gemeinsamen Längsachse (L) eine entsprechende Längsdehnung (Δl) einer schlauchartigen Struktur (32) des Elektrodenfixierelements (30) erzeugbar ist und infolge eines definierten Querkontraktionsverhaltens der schlauchartigen Struktur (32) ein Haftschluss zwischen der schlauchartigen Struktur (32) und der Elektrodenleitung (14) herstellbar ist.

14. System (10) nach Anspruch 13, **dadurch gekennzeichnet, dass** der Haftschluss eine Haftreibungskraft (Fᵣ) von 2 N bis 50 N zu übertragen vermag.

15. System (10) nach Anspruch 14, **dadurch gekennzeichnet, dass** der Haftschluss durch einen relativen Verstellweg (s) von 2 mm bis 20 mm herstellbar ist.
